# EUROPÄISCHE PATENTANMELDUNG

(11) **EP 3 909 564 A1**
(43) Veröffentlichungstag der Anmeldung: **17.11.2021**
(21) Anmeldenummer: 20174201.2
(22) Anmeldetag: 12.05.2020
(51) Int. Cl.: A61K 9/00, A23L 2/39, A61K 9/14, A61K 31/122, A61K 47/46

(54) **FESTE LÖSUNG AUS GUMMI ARABICUM UND WENIGSTENS EINEM FETTLÖSLICHEN WIRKSTOFF**

(71) Anmelder: Metabolic Tuning AG, 9327 Tübach (CH)
(72) Erfinder: STROBEL, Hanspeter, 7270 Davos Platz (CH)
(74) Vertreter: Schneiders & Behrendt Bochum

(57) **Zusammenfassung**

Feste Lösung aus Gummi arabicum und wenigstens einem fettlöslichen Wirkstoff mit Teilchen einer Größe von weniger als 100 µm und einem Gewichtsverhältnis von Gummi arabicum zu fettlöslichen Wirkstoffen von 5:1 bis 200:1, erhältlich durch Trocknen einer wässrigen Lösung von Gummi arabicum zu einem feinteiligen Trockenprodukt; Versetzen des Trockenprodukts mit einer Lösung oder Dispersion des oder der fettlöslichen Wirkstoffe in einem wasserfreien Lösungsmittel, in dem Gummi arabicum nicht löslich ist; Einmischen der Lösung unter Homogenisierung und weiterer Zerteilung des Trockenprodukts auf eine Teilchengröße von weniger als 100 µm; und Entfernen des Lösungsmittels bei einer Temperatur von weniger als 70°C sowie Verfahren zu ihrer Herstellung und diese enthaltende wässrige Suspension.

## Beschreibung

Die Erfindung betrifft eine feste Lösung aus Gummi arabicum und wenigstens einem fettlöslichen Wirkstoff mit Teilchen einer Größe von weniger als 100 µm und einem Gewichtsverhältnis von Gummi arabicum zu fettlöslichem Wirkstoff von 5:1 bis 200:1 sowie ein Verfahren zur Herstellung solcher festen Lösungen und diese enthaltende wässrige Suspensionen.

Die orale Verabreichung von fettlöslichen Wirkstoffen ist häufig ein Problem, weil die Wirkstoffe aus einer wässrigen Umgebung, wie sie im Darm und auf Schleimhäuten vorherrschen, vom Körper nicht resorbiert werden. Auch bei der Verabreichung mit einem lipophilen Träger bleibt die Aufnahmerate häufig hinter den Erwartungen zurück.

Zur Verbesserung der Aufnahme von Wirkstoffen im Körper wurde deshalb vorgeschlagen, die Wirkstoffe eingebunden in Mizellen zu verabreichen. Mizellen werden gebildet von Stoffen, die über ein lipophiles und ein hydrophiles Ende verfügen und damit in einer wässrigen Umgebung lipophile Stoffe einschließen und transportieren können. Beispiele sind hierfür beispielsweise eine Vielzahl von waschaktiven Substanzen (Detergenzien), Polysorbate, Poloxamere sowie eine Vielzahl von Naturstoffen, darunter eine Reihe von natürlichen Harzen, auch Gummi arabicum.

In der WO 2007/104173 A2 ist die Verwendung von Poloxameren zur Sulubilisierung von Wirkstoffen, darunter Insulin, beschrieben. Hingewiesen wird dabei auch auf die grundsätzliche Eignung von Harzen für diese Zwecke.

Die WO 2008/034273 A1 beschreibt Zusammensetzungen auf Basis von Gummi arabicum, das in Form von Mizellen mit einem darin gebundenen Wirkstoff vorliegt, wobei die Zusammensetzungen aus einer warmen wässrigen Lösung erhalten werden. Die Zusammensetzung ist flüssig und weist Mizellen mit einem Durchmesser von 2 bis 300 nm auf.

Allen diesen Ansätzen ist gemein, dass sie die Aufnahme des jeweiligen Wirkstoffs in den Körper nicht grundsätzlich verbessern.

Der Erfindung liegt die Aufgabe zugrunde, ein Mittel bereitzustellen, mit dem die Aufnahme fettlöslicher Wirkstoffe in den Körper effizienter gestaltet werden kann. Gleichzeitig soll ein Verfahren zur Herstellung dieses Mittels aufgezeigt werden und sollen Beispiele für die Verabreichung dieses Mittels gegeben werden.

Die Teilchengröße der aus Gummi arabicum und den Wirkstoffen gebildeten Partikel hat einen Einfluss auf die Verabreichungsform wie auf die Verteilung des oder der Wirkstoffe. Die Teilchengröße wird zum einen bestimmt durch das Herstellungsverfahren und zum anderen durch das Verhältnis von Gummi arabicum zu Wirkstoff.

Gummi arabicum hat eine suspendierende und emulsionsstabilisierende Wirkung. Es bildet um fettlösliche Substanzen einen Schutzkolloidüberzug, der zur Ausbildung einer festen Lösung aus Gummi arabicum und fettlöslichen Wirkstoffen genutzt werden kann und deren Transport und Aufnahme durch einen Organismus erleichtert.

Entsprechend betrifft die Erfindung eine feste Lösung aus Gummi arabicum und wenigstens einem fettlöslichen Wirkstoff mit Teilchen einer Größe von weniger als 100 µm und einem Gewichtsverhältnis von Gummi arabicum zu fettlöslichen Wirkstoffen von 20:1 bis 200:1, erhältlich durch Trocknen einer wässrigen Lösung von Gummi arabicum zu einem feinteiligen Trockenprodukt; Versetzen des Trockenprodukts mit einer Lösung des oder der fettlöslichen Wirkstoffe in einem wasserfreien Lösungsmittel, in dem Gummi arabicum nicht löslich ist, Einmischen der Lösung unter Homogenisierung und weiterer Zerteilung des Trockenprodukts auf eine Teilchengröße von weniger als 100 µm; und Entfernen des Lösungsmittels bei einer Temperatur unterhalb der Zersetzungstemperaturen von Gummi arabicum, insbesondere bei weniger als 70° C.

Die Erfindung betrifft ferner ein Verfahren zur Herstellung einer festen Lösung aus Gummi arabicum und wenigstens einem fettlöslichen Wirkstoff, gekennzeichnet durch die Schritte: Trocknen einer wässrigen Lösung von Gummi arabicum zu einem feinteiligen Trockenprodukt; Versetzen des feinteiligen Trockenprodukts mit einer wasserfreien Lösung oder Dispersion des oder der fettlöslichen Wirkstoffe in einem Lösungsmittel, in dem Gummi arabicum nicht löslich ist, in einem Gewichtsverhältnis von Gummi arabicum zu fettlöslichen Wirkstoffen von 20:1 bis 200:1; Einmischen der Lösung unter Homogenisierung und weiterer Zerteilung des Trockenprodukts auf eine Teilchengröße kleiner 100 µm; und Entfernen des Lösungsmittels bei einer Temperatur ≤ 70° C, vorzugsweise ≤ 40° C, insbesondere Raumtemperatur.

Für das Verständnis wesentlich ist, dass die genannten Teilchengrößen Ausschlussgrenzen sind. Entsprechend bedeutet "Teilchen einer Größe von weniger als 100 µm", dass die Teilchen weit überwiegend kleiner als 100 µm sind. Es handelt sich nicht um eine mittlere Teilchengröße einer statistischen Verteilung. Dies gilt sowohl für die unteren als auch für die oberen Ausschlussgrenzen.

Wesentlich ist ferner, dass das Herstellungsverfahren bei einer Temperatur ≤ 70° C, vorzugsweise ≤ 40° C und insbesondere bei Raumtemperatur durchgeführt wird. Aus der Lösung oder Schmelze wird die erforderliche Feinteiligkeit nicht erreicht. Gummi arabicum kann sich in Gegenwart von wenig Wasser bei einer Temperatur von 40°C bis 60°C lösen. Die Löslichkeit von Gummi arabicum in Wasser beträgt etwa 500 g/l.

Die erfindungsgemäße feste Lösung aus Gummi arabicum und dem oder den Wirkstoffen liegt in wässrigem Medium in Form von Mizellen vor. Mizellen als Transportmittel für fettartige Wirkstoffe werden seit Jahrzehnten zur Sulubilisierung von wasserunlöslichen Stoffen eingesetzt. Letztlich basiert die Reinigungswirkung von Tensiden auf der Bildung von Mizellen.

Mizellen sind Aggregate aus Tensidmolekülen, deren Inneres in wässriger Umgebung aus lipophilen Gruppen der einzelnen Tensidmolekülen besteht, während die hydrophilen Gruppen an der Oberfläche sitzen. Diese hydrophilen Gruppen sind zur Wasseranlagerung befähigt und führen zur Ausbildung einer Emulsion.

In ihrer einfachsten Form haben die Mizellen Kugelgestalt, zu der sie sich spontan selbst organisieren. Die verbliebenen Reste im Inneren sind nicht in Kontakt mit der umgebenden wässrigen Phase. Der Durchmesser der Mizellen ist gegeben durch den Platzbedarf des lipophilen Kerns und der umgebenden hydrophilen Hülle. Man kann sie als Flüssigkeitstropfen in ihrer jeweiligen Umgebung ansehen.

Bei einem Überangebot an Tensidmolekülen steigen die Durchmesser der Aggregate deutlich an und erreichen Werte, die weit über dem zehnfachen des kleinstmöglichen Durchmessers liegen.

Es ist deswegen wesentlich, das Verhältnis von Gummi arabicum zu dem oder den Wirkstoffen im erfindungsgemäßen Bereich zu halten, da dies die Partikelgröße der gebildeten Mizellen begrenzt.

In einer Mizelle können die einzelnen Tensidmoleküle in wässriger Umgebung jederzeit aus dem Mizellverband in die umgebende Phase austreten und umgekehrt freie Tensidmoleküle sich mit Wirkstoffen zu neuen Mizellen zusammenfinden. Es findet ein steter Austausch statt. Dieses Gleichgewicht zwischen Mizelle und freien Tensid- und Wirkstoffmolekülen ermöglicht an einer durchlässigen Membran den Transfer des Wirkstoffs in und durch die Membran.

Die Transportwirkung der erfindungsgemäßen Partikel aus Gummi arabicum und fettlöslichem Wirkstoff beruht also darauf, dass der Wirkstoff in wässriger Umgebung von der Mizelle an den Ort des Transfers transportiert und dort freigesetzt wird. Mit der Freisetzung tritt der Wirkstoff durch die Membranin die Zellen ein.

Gummi arabicum wird aus dem Pflanzensaft von verschiedenen Akazien (beispielsweise Acacia senegal, Acacia seyal) gewonnen und besteht hauptsächlich aus den sauren Alkali- und Erdalkalisalzen der Arabinsäure (Polyarabinsäure). Polyarabinsäure ist ein verzweigtes, aus L-Arabinose, D-Galaktose, L-Rhamnose und D-Glucuronsäure im Verhältnis von 3:3:1:1 bestehendes Polysaccharid mit einem Molekulargewicht im Bereich von 200.000 bis 350.000 g/mol, je nach Messverfahren.

Es wird in der Lebensmittelindustrie als Verdickungsmittel, Emulgator und Stabilisator eingesetzt. Es findet weiter Anwendung in der Getränkeindustrie, bei Süßwaren, in der Pharmazie, in der Kosmetik sowie in Bereichen der Technik.

Erfindungsgemäß können grundsätzlich alle Gummi arabicum-Sorten verwandt werden. Voraussetzung ist eine gewisse Feinteiligkeit bereits vor der erfindungsgemäßen Verarbeitung, die insbesondere bei Sprühtrocknung aus einer wässrigen Lösung vorliegt. Die Teilchengröße nach Sprühtrocknung ist allerdings für erfindungsgemäße Zwecke noch nicht geeignet, weil zu grob. Durch Sprühtrocknung erhaltenes Gummi arabicum-Pulver ist in guter Qualität kommerziell mit einer Schüttdichte von 600 kg/m3 und eine innere spez. Oberfläche von ≥ 150 m²/g erhältlich. Diese Kapillarität des wasserfesten Pulvers steht für die Einlagerung des oder der lipophilen Wirkstoffe zur Verfügung. Die weitere Zerkleinerung führt dann zur Ausbildung der erfindungsgemäßen Partikel.

Das zum Einsatz kommende Gummi arabicum-Pulver sollte deshalb eine amorphe Pulverstruktur mit vielen Kapillaren und großer innerer Oberfläche aufweisen. Die große innere Oberfläche ist wesentlich für das "Aufsaugen" der Wirkstofflösung oder -dispersion und ihre möglichst gleichmäßige Verteilung. Gleichzeitig ist eine gute Wasserlöslichkeit essenziell, die aber regelmäßig bei durch Sprühtrocknung erhaltenem Produkt gegeben ist. Damit lassen sich hochkonzentrierte Lösungen oder Suspensionen verhältnismäßig niedriger Viskosität herstellen.

Das erfindungsgemäße Herstellungsverfahren wird unter Ausschluss von Wasser durchgeführt; die erfindungsgemäße feste Lösung ist bei Erhalt wasserfrei. Dies ist wesentlich für die Stabilität des Produkts wie auch für die Brauchbarkeit daraus erhaltener wässriger Suspensionen und Lösungen.

Das erfindungsgemäße Verfahren wie auch die danach erhaltene feste Lösung ist geeignet, diese Mizellen über Jahre in einer "solid solution" zu stabilisieren. Dabei sind die gebildeten Partikel (Mizellen) selbst dann, wenn der Wirkstoff in flüssiger Form vorliegt, fest und bilden ein trockenes Pulver, welches als "Halbfabrikat" in jede galenische Form (Creme, Serum, Lotion, Gel, Getränk, shot) problemlos eingearbeitet werden kann. Das Pulver kann ohne Schwierigkeiten in geringen Mengen Wasser oder einer wässrigen Lösung suspendiert werden - erst bei Unterschreiten der Löslichkeitsgrenze tritt vollständige Emulsion auf.

Durch den Einsatz verschiedener Wirkstoff-Module kann einfach, ökonomisch und ohne großen Entwicklungsaufwand eine Vielzahl von Wirkungen erzeugt werden. Die Wirkungen solcher Wirkstoff-Komplexe tritt dabei schneller ein als bei üblichen "Carrier-Systemen".

Gummi arabicum und Wirkstoff werden erfindungsgemäß in einem Gewichtsverhältnis von 5:1 bis 200:1, insbesondere in einem Gewichtsverhältnis von 10:1 bis 100:1 eingesetzt. Das Gewichtsverhältnis ist von Bedeutung, weil ein zu hoher Anteil von Gummi arabicum zu einer Vergrößerung der Partikel führen kann, die einer wirkungsvollen Verteilung entgegensteht.

Wie schon angedeutet, ist die Teilchengröße von wesentlicher Bedeutung. Die Partikel der festen Lösung sollten alle im Wesentlichen ≤ 100 µm sein, vorzugsweise zu einem Anteil von 80 % ≤ 60 µm. Gleichzeitig sollte zur Erzielung eines homogenen Produkts die Teilchengröße nach unten begrenzt sein, dergestalt, dass insbesondere 80 % der Partikel eine Teilchengröße ≥ 1 µm und insbesondere 90 % der Partikel eine Teilchengröße von ≥ 1 µm aufweisen. Die mittlere Teilchengröße dürfte dabei in etwa bei 40 µm liegen.

Für die Einbringung des oder der Wirkstoffe in Lösung ist die Feinteiligkeit und innere Oberfläche des zum Einsatz kommenden Matrixmaterials aus Gummi arabicum entscheidend. Die Lösung muss vollkommen und möglichst gleichmäßig aufgenommen werden und sich im Inneren des Matrixmaterials verteilen. Wichtig ist dabei, dass das Lösungsmittel für den oder die Wirkstoffe das Gummi arabicum nicht löst und damit seine Struktur nicht verändert.

Geeignete Lösungsmittel sind solche, die den jeweiligen lipophilen Wirkstoff auflösen und möglichst hochkonzentrierte Lösungen bilden. Das Lösungsmittel soll wasserfrei sein. Ein weiteres Kriterium ist ein möglichst niedriger Siedepunkt, der die Entfernung des Lösungsmittels aus dem Produkt erlaubt. Geeignete Lösungsmittel sind beispielsweise Ether und Alkohole, insbesondere Ethanol. Besonders bevorzugt ist absoluter Alkohol (100 %). Die Gegenwart von Wasser im mit Wirkstoff versetzten Matrixmaterial ist zu vermeiden.

Wesentliche Voraussetzung für die Erzeugung einer erfindungsgemäßen festen Lösung ist das Homogenisieren und die weitere Zerteilung des mit dem oder den Wirkstoffen imprägnierten Matrixmaterials. Hierzu kann im Grunde genommen jedes Verfahren eingesetzt werden, das die richtigen Teilchengrößen gewährleistet. Besonders bevorzugt für diese Zwecke ist der Einsatz von Cuttern und Homogenisierern mit hoher Scherkraft.

Bei der Homogenisierung oder in einem letzten Schritt des Herstellungsverfahrens wird das Lösungsmittel des oder der Wirkstoffe entfernt. Bei leicht flüchtigen Lösungsmitteln, wie Alkohol, verflüchtigt sich ein Großteil des Lösungsmittels bereits während der Homogenisierung und weiteren Zerteilung. Den verbleibenden Rest kann man bei einer Temperatur ≤ 70° C, vorzugsweise ≤ 40° C und insbesondere bei Raumtemperatur abdunsten lassen oder auch im Vakuum abziehen.

Bei dieser Vorgehensweise wird ein stabiles Pulver aus Gummi arabicum und dem oder den Wirkstoffen erhalten. Dieses Pulver ist ohne weiteres in kleinen Mengen Wasser suspendierbar und größeren Wassermengen löslich. Die wässrigen Suspensionen oder Lösungen sind ebenfalls stabil; es erfolgen keine Agglomerationen.

Die erfindungsgemäße feste Lösung enthält, eingeschlossen im Gummi arabicum, vorzugsweise nur einen fettlöslichen Wirkstoff. Es versteht sich aber, dass auch Gemische erfasst werden, die zum einen dadurch erhalten werden können, dass in einem Herstellungsverfahren gleichzeitig mehrere fettlösliche Wirkstoffe in die Gummi arabicum-Partikel eingebracht werden oder feste Lösungen mehrerer Wirkstoffe miteinander vermischt werden. Entsprechendes gilt für das Herstellungsverfahren.

Die erfindungsgemäßen festen Lösungen können als Pulver vermarktet werden, dienen aber vor allem als Ausgangsstoff für Suspensionen, die 2,5 bis 40,0 Gewichtsprozent der festen Lösung suspendiert in Wasser enthalten. Derartige Suspensionen können zusätzlich übliche lebensmitteltaugliche Zusatzstoffe enthalten, etwa Konservierungsmittel, Stabilisatoren, pH-Regulatoren, Süßungsmittel, Aromastoffe.

Weiterhin können wasserlösliche Vitamine, Provitamine, Nahrungsergänzungsmittel und Nährstoffe in üblichen Mengen zugesetzt sein. Übliche Mengen sind in diesem Zusammenhang 30 - 300 % einer empfohlenen Tagesdosis. Der gesamte Gehalt dieser Zusätze beläuft sich in der Regel auf 0,5 - 2,5 Gewichtsprozent der wässrigen Suspension.

Es versteht sich, dass in derartigen wässrigen Suspensionen ein Teil der zugesetzten Partikel aus Gummi arabicum und fettlöslichem Wirkstoff in Lösung geht, je nach Konzentration. In einer fünfprozentigen wässrigen Suspension liegt in aller Regel ein Teil des Gummi arabicums mit eingeschlossenem Wirkstoff in Partikelform vor; ein Teil eines Films oder Schwammes.

Als fettlöslicher Wirkstoff kommen grundsätzlich alle Wirkstoffe infrage, die in gelöster Form gemäß erfindungsgemäßem Verfahren in das feinteilige Gummi arabicum eingebracht werden können. Zu nennen sind beispielsweise Coenzym Q10, Coenzym Q10H2, Vitamin A, Vitamin D, Vitamin E (Tocopherol), Vitamin K, fettlösliche Vitamine der B-Gruppe, Curcumin, Chalcone, Flavan-3-ole, Flavanone, Flavone, Flavonole, Isoflavone, Flavanolole, essenzielle Fettsäuren, Wachstumsfaktoren, Immunglobuline, Immunregulatoren, Hormone (Progesteron, Testosteron, Estron, Estradiol, Androstendion, etc.) sowie zahlreiche fettlösliche Arzneimittel (Analgetika, entzündungshemmende Mittel, Antibiotika).

### Beispiel

Als Wirkstoff wurde CoEnzym Q10 (Ubichinon) verwendet. Dazu wurden 100 g durch Sprühtrocknung erhaltenes Gummi arabicum-Pulver mit 2,0 g Ubichinon suspendiert in 10 ml absolutem Alkohol versetzt und in einem Cutter (Robocoupe) 90 Sek. homogenisiert und auf eine Teilchengröße von ≤ 100 µm zerkleinert. Das erhaltene feinteilige Pulver der festen Lösung von Ubichinon in Gummi arabicum wurde bei Raumtemperatur unter Lichtschutz stehen gelassen, bis der Alkohol verdunstet war. Das Pulver ist bei kühler Lagerung unter Lichtschutz stabil.

51 g des so erhaltenen Pulvers wurden zusammen mit 0,5 g Zitronensäure (pH-Regulator), 1 g Ascorbinsäure (Stabilisator) und 1 g Kaliumsorbat (Konservierungsmittel) in 946,5 g destilliertes Wasser gegeben, woraus sich eine über Monate stabile Suspension ergab. Diese Suspension ist trinkfertig und unmittelbar zur oralen Einnahme des Ubichinons geeignet.

Der so erhaltenen Suspension können weitere Stoffe zugesetzt werden, etwa wasserlösliche Vitamine, Provitamine, Nahrungsergänzungsmittel, Nährstoffe, Süßungsmittel und Aromen. Als insbesondere geeignet hat sich der Zusatz von Vitaminen, Kurkumaextrakt (Curcumin) und Passionsfruchtextrakt (Chrysin) erwiesen. Diese und andere Zusätze können als Escort-Hilfe dienen.

Natriumhydrogencarbonat (etwa 4 g/l) kann als Trennmittel zugesetzt werden. Es hat sich gezeigt, dass der Zusatz die Auflösung des Pulvers beschleunigt, in Verbindung mit Vitamin C auch ein Aufschäumen bewirkt.

## Patentansprüche

1. Feste Lösung aus Gummi arabicum und wenigstens einem fettlöslichen Wirkstoff mit Teilchen einer Größe von weniger als 100 µm und einem Gewichtsverhältnis von Gummi arabicum zu fettlöslichen Wirkstoffen von 5:1 bis 200:1, erhältlich durch
Trocknen einer wässrigen Lösung von Gummi arabicum zu einem feinteiligen Trockenprodukt;
Versetzen des Trockenprodukts mit einer Lösung oder Dispersion des oder der fettlöslichen Wirkstoffe in einem wasserfreien Lösungsmittel, in dem Gummi arabicum nicht löslich ist;
Einmischen der Lösung unter Homogenisierung und weiterer Zerteilung des Trockenprodukts auf eine Teilchengröße von weniger als 100 µm; und
Entfernen des Lösungsmittels bei einer Temperatur ≤ 70° C, vorzugsweise bei ≤ 40°C

2. Feste Lösung nach Anspruch 1, **dadurch gekennzeichnet, dass** die Teilchen aus Gummi arabicum und dem wenigstens einen fettlöslichen Wirkstoff Mizellen ausbilden.

3. Feste Lösung nach Anspruch 1 oder 2, **dadurch gekennzeichnet, dass** das Gewichtsverhältnis von Gummi arabicum zu fettlöslichen Wirkstoffen im Bereich von 50:1 bis 100:1 liegt.

4. Feste Lösung nach einem der vorstehenden Ansprüche, **dadurch gekennzeichnet, dass** 80 % der Teilchen, eine Größe von ≤ 60 µm haben.

5. Feste Lösung nach Anspruch 4, **dadurch gekennzeichnet, dass** 80 % der Teilchen eine Größe von ≤ 40 µm haben.

6. Feste Lösung nach einem der vorstehenden Ansprüche, **dadurch gekennzeichnet, dass** das feinteilige Trockenprodukt sprühgetrocknetes Gummi arabicum ist.

7. Feste Lösung nach einem der vorstehenden Ansprüche, **dadurch gekennzeichnet, dass** das Lösungsmittel der Lösung oder Dispersion des oder der fettlöslichen Wirkstoffe absoluter Alkohol ist.

8. Verfahren zur Herstellung einer festen Lösung aus Gummi arabicum und wenigstens einem fettlöslichen Wirkstoff, **gekennzeichnet durch** die Schritte
Trocknen einer wässrigen Lösung von Gummi arabicum zu einem feinteiligen Trockenprodukt;
Versetzen des feinteiligen Trockenprodukts mit einer Lösung oder Dispersion des oder der fettlöslichen Wirkstoffe in einem wasserfreien Lösungsmittel, in dem Gummi arabicum nicht löslich ist, in einem Gewichtsverhältnis von Gummi arabicum zu fettlöslichen Wirkstoffen von 20:1 bis 200:1;
Einmischen der Lösung unter Homogenisierung und weiterer Zerteilung des Trockenprodukts auf eine Teilchengröße kleiner 100 µm; und
Entfernen des Lösungsmittels bei einer Temperatur ≤ 70° C, vorzugsweise ≤ 40° C.

9. Verfahren nach Anspruch 8, **dadurch gekennzeichnet, dass** als Trockenprodukt sprühgetrocknetes Gummi arabicum verwandt wird.

10. Verfahren nach Anspruch 8 oder 9, **dadurch gekennzeichnet, dass** die weitere Zerteilung mit einem Cutter oder Sonicator vorgenommen wird.

11. Verfahren nach einem der Ansprüche 8 bis10, **dadurch gekennzeichnet, dass** die weitere Zerteilung unter hoher Scherwirkung vorgenommen wird, bis 80 % der Teilchen eine Größe von ≤ 60 µm aufweisen.

12. Verfahren nach Anspruch 11, **dadurch gekennzeichnet, dass** das die weitere Zerteilung vorgenommen wird, bis 80 % der Teilchen eine Teilchengröße von ≤ 40 µm aufweisen.

13. Verfahren nach einem der Ansprüche 8 bis 12, **dadurch gekennzeichnet, dass** ein Gewichtsverhältnis von Gummi arabicum zu fettlöslichen Wirkstoffen von 1:50 bis 1:100 verwandt wird.

14. Verfahren nach einem der Ansprüche 8 bis 13, **dadurch gekennzeichnet, dass** als Lösungsmittel absoluter Alkohol verwandt wird.

15. Wässrige Suspension, enthaltend 2,0 bis 40,0 Gew.-% der festen Lösung aus Gummi arabicum nach einem der Ansprüche 1 bis 8.

16. Wässrige Suspension nach Anspruch 15, **dadurch kennzeichnet, dass** sie zusätzlich übliche lebensmitteltaugliche Konservierungsmittel, Stabilisatoren, pH-Regulatoren, Süßungsmittel und oder Aromastoffe in einer Menge von, zusammengenommen, 0,05 bis 1,0 Gew.-% enthält.

17. Wässrige Suspension nach Anspruch 15 oder 16, **dadurch gekennzeichnet, dass** sie zusätzlich wasserlösliche Vitamine, Provitamine, Nährstoffe und/oder Nahrungsergänzungsmittel in einer Menge von, zusammengenommen, 0,5 bis 2,5 Gew.-% enthält.

18. Feste Lösung nach einem der Ansprüche 1 bis 7, Verfahren nach einem der Ansprüche 8 bis 13 und wässrige Suspension nach einem der Ansprüche 15 bis 17, **dadurch gekennzeichnet, dass** der oder einer der Wirkstoffe CoEnzym Q10 und/oder Curcumin ist.
